(19) Europäisches Patentamt
European Patent Office
Office européen des brevets

(11) Veröffentlichungsnummer: **0 217 159 B1**

# (12) EUROPÄISCHE PATENTSCHRIFT

(45) Veröffentlichungstag der Patentschrift: **02.01.92**

(51) Int. Cl.5: **C07C 33/20**, C07C 29/54, C07C 47/228, C07C 45/68, C07C 29/40, C07C 43/164, C07C 41/16, C07C 29/14, A61K 7/46, C07C 29/143

(21) Anmeldenummer: **86112147.3**

(22) Anmeldetag: **02.09.86**

Die Akte enthält technische Angaben, die nach dem Eingang der Anmeldung eingereicht wurden und die nicht in dieser Patentschrift enthalten sind.

## (54) Aldehyde, Acetale, Alkohole und Ether mit 3-Methyl- oder 3,5-Dimethylbenzylgruppen, deren Herstellung und ihre Verwendung als Duftstoffe.

(30) Priorität: **04.09.85 DE 3531585**

(43) Veröffentlichungstag der Anmeldung:
**08.04.87 Patentblatt 87/15**

(45) Bekanntmachung des Hinweises auf die Patenterteilung:
**02.01.92 Patentblatt 92/01**

(84) Benannte Vertragsstaaten:
**AT BE CH DE FR IT LI NL**

(56) Entgegenhaltungen:
**EP-A- 0 032 659**
**EP-A- 0 044 514**
**US-A- 4 490 284**

**LIEBIGS ANNALEN DER CHEMIE, Band 751, 1971, Seiten 109-120, Verlag Chemie, Weinheim, DE; H. KROPF et al.: "Organische Peroxide VII"**

(73) Patentinhaber: **Consortium für elektrochemische Industrie GmbH**
**Zielstattstrasse 20**
**W-8000 München 70(DE)**

(72) Erfinder: **Hafner, Walter, Dr. Dipl.-Chem.**
**Birkenallee 17**
**W-8196 Eurasburg(DE)**
Erfinder: **Ritter, Peter**
**Brotkorbweg 60**
**W-8960 Kempten(DE)**
Erfinder: **Gebauer, Helmut, Dr. Dipl.-Chem.**
**Schaffhauser Strasse 18/7**
**W-8000 München 71(DE)**
Erfinder: **Regiert, Marlies**
**Schraudolphstrasse 2a**
**W-8000 München 40(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Auswahl von Aldehyden, davon abgeleiteten Acetalen, Alkoholen und davon abgeleiteten Ethern mit 3-Methylbenzyl- oder 3.5-Dimethylbenzyl-Substituenten.

Gemäß Chemikerzeitung 97 (1973), Nr. 1, Seite 8 ff., ist bereits Benzyldimethylcarbinol als Duftstoff mit blumiger Note bekanntgeworden.

Weiter wird in US-A-4,490,284 die Verwendung von 1,1-Di-($C_1$-$C_6$-alkyl)-2-phenylethan-Derivaten als Duftstoffkomponenten geschützt. Besonders der 2,2-Dimethyl-3-phenylpropionaldehyd und der entsprechende Alkohol

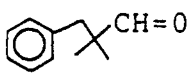

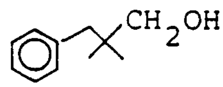

werden als Verbindungen mit starkem aldehydisch-grünem Geruch, bzw. mit starkem blumigem Duft, erinnernd an Maiglöckchen, Hyacinthen und gewisse tertiäre Carbinole beschrieben. Dazu wurden Anwendungsbeispiele in Duftstoffkompositionen gebracht.

Es wurde nun gefunden, daß sich eine Auswahl von 3- bzw. 3.5-Dimethylbenzylverbindungen gegenüber den analogen nicht substituierten Verbindungen durch verbesserte Duftstoffqualität auszeichnet: Die das natürliche Geruchsbild störenden Nebennoten treten zurück; das Geruchsspektrum verdichtet sich zu einer um so intensiveren, natürlichen Note.

Gegenstand der Erfindung sind Verbindungen der allgemeinen Formel (I)

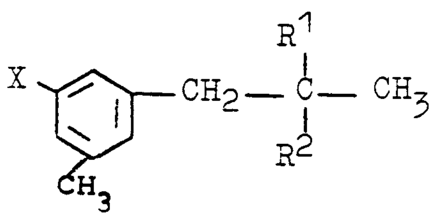

und Verbindungen der Formel (II)

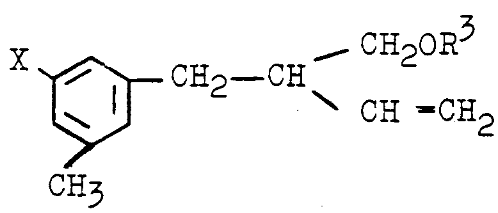

wobei

- $R^1$ für -OH, $-CH_2OH$, $CH_2$-$OCH_3$, -CHOH-$CH_3$, -CHO und $-CH(OCH_3)_2$ steht
- $R^2$ Methyl oder Ethyl
- $R^3$ Wasserstoff oder Methyl und
- X Wasserstoff oder Methyl

bedeuten und 2-Methyl-3-(3-methylphenyl)-propan-2-ol und 2-Methyl-3-(3,5-dimethylphenyl)-propan-2-ol ausgeschlossen sind.

Beispiele für erfindungsgemäße Verbindungen sind:

2-(3-Methylbenzyl)-3-buten-1-ol
2-(3.5-Dimethylbenzyl)-3-buten-1-ol
1-Methoxy-2-(3-methylbenzyl)-3-buten
1-Methoxy-2-(3.5-dimethylbenzyl)-3-buten

2.2-Dimethyl-3-(3-methylphenyl)-propan-1-ol
2.2-Dimethyl-3-(3.5-dimethylphenyl)-propan-1-ol
2-Methyl-2-(3-methylbenzyl)-butan-1-ol
2-Methyl-2-(3.5-dimethylbenzyl)-butan-1-ol
2-(3-methylbenzyl)-propan-2-ol
2-(3.5-dimethylbenzyl)-propan-2-ol
2-(3-methylbenzyl)-butan-2-ol
2-(3.5-dimethylbenzyl)-butan-2-ol
2.2-Dimethyl-3-(3-methylphenyl)-propionaldehyd
2.2-Dimethyl-3-(3.5-dimethylphenyl)-propionaldehyd
2-Methyl-2-(3-methylbenzyl)-butyraldehyd
2-Methyl-2-(3.5-dimethylbenzyl)-butyraldehyd
1.1-Dimethoxy-2.2-dimethyl-3-(3-methylphenyl)-propan
1.1-Dimethoxy-2.2-dimethyl-3-(3.5-dimethylphenyl)-propan
1.1-Dimethoxy-2.2-dimethyl-3-(3-methylphenyl)-butan
1.1-Dimethoxy-2.2-dimethyl-3-(3.5-dimethylphenyl)-butan
3-Methyl-3-(3-methylbenzyl)-butan-2-ol
3-Methyl-3-(3.5-dimethylbenzyl)-butan-2-ol
3-Methyl-3-(3-methylbenzyl)-pentan-2-ol
3-Methyl-3-(3.5-dimethylbenzyl)-pentan-2-ol
1-Methoxy-2.2-dimethyl-3-(3-methylphenyl)-propan
1-Methoxy-2.2-dimethyl-3-(3.5-dimethylphenyl)-propan.

Die Verbindungen der Formel II sind aus Butadienmagnesium und den entsprechenden Benzylchloriden bei nachfolgender Oxidation und anschließender Hydrolyse zugänglich. Gegebenenfalls schließt sich noch n einer weiteren Reaktionsstufe die Veretherung der freien OH-Gruppe an.

Das Verfahren ist dadurch gekennzeichnet, daß

a) Benzylchlorid der Formel

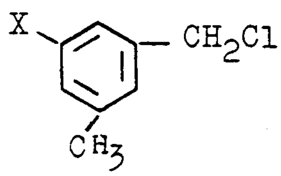

wobei

X für Wasserstoff oder Methyl steht

mit Butadienmagnesium umgesetzt wird,

b) das Reaktionsprodukt gemäß a) oxidiert wird,

c) das Oxidationsprodukt gemäß b) einer sauren Hydrolyse unterworfen wird,

d) das Reaktionsprodukt gemäß c) ggf. an der freien OH-Gruppe methyliert wird.

Der Reaktionsweg wird an der folgenden Gleichung verdeutlicht:

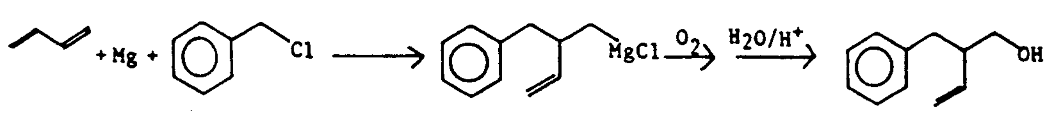


Butadienmagnesium ist in an sich bekannter Weise aus metallischem Magnesium und Butadien erhältlich. In der Regel werden Lösungsmittel wie beispielsweise Tetrahydrofuran u. a. verwendet.

Die als Ausgangssubstanzen verwendeten Benzylchloride sind ebenfalls bekannte Substanzen. Die Umsetzung der genannten Ausgangsverbindungen erfolgt zweckmäßigerweise bei Temperaturen von 0 °C bis 65 °C. Die Oxidation des erhaltenen Magnesiumchlorids erfolgt am besten mit Sauerstoff oder sauerstoffhaltigen Gasen, insbesondere Luft. Die Oxidationstemperaturen betragen zumeist -25°C bis 30 °C.

Die Hydrolyse des Oxidationsprodukts erfolgt am besten in Gegenwart von Mineralsäuren, wie Salzsäure, Schwefelsäure und dgl.

Gegebenenfalls erfolgt noch die Veretherung der freien OH-Gruppe nach an sich bekannten Methoden:

Beispielsweise wird der Alkohol in des Alkoholat übergeführt und anschließend mit Methylierungsmitteln, wie Dimethylsulfat und dgl., zum entsprechenden Ether umgesetzt.

Die Verbindungen der Formel I sind, soweit $R^1$ einen kohlenstoffhaltigen Rest bedeutet, durch Kondensation der entsprechenden Benzylchloride mit Isobutyraldehyd bzw. mit 2-Methylbutyraldehyd und ggf. weiterer Derivatisierung der erhaltenen Aldehyde zugänglich.

Das Verfahren zur Herstellung der Verbindungen der Formel

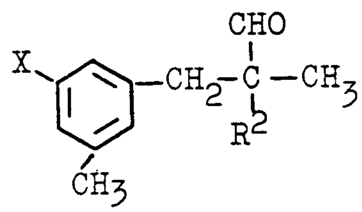

wobei

$R^2$ Methyl oder Ethyl und

X Wasserstoff oder Methyl

bedeuten,

ist dadurch gekennzeichnet, daß Benzylchloride der Formel

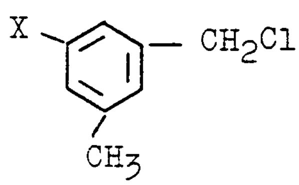

mit Isobutyraldehyd oder 2-Methylbutyraldehyd in einem organisch/alkalischen 2-Phasensystem ggf. in Gegenwart von Phasentransferkatalysatoren kondensiert werden.

Benzylchlorid und Aldehyd werden in etwa äquimolaren Mengen eingesetzt. Das organisch/alkalische 2-Phasensystem wird gebildet aus einem organischen, mit Wasser nicht mischbaren inerten Lösungsmittel und einer 5 bis 50 Gew.-%igen wäßrigen Lösung oder in fester Form vorliegendem Alkalimetallhydroxid.

Als Phasentransferkatalysatoren werden beispielsweise Kronenether, quartäre Ammonium- und Phosphoniumsalze eingesetzt in Mengen von 0,5 bis 5 Mol-%, bezogen auf Benzylchlorid.

Die Reaktionstemperaturen betragen 20°C bis 150°C, insbesondere 60°C bis 70°C.

Zweckmäßigerweise wird das Verfahren so durchgeführt, daß das 2-Phasensystem mit dem Katalysator vorgelegt und ein Gemisch der Reaktionskomponenten zugetropft wird.

Aus den erhaltenen Aldehyden sind die entsprechenden primären Alkohole durch an sich bekannte Reduktionsverfahren zugänglich. Die sekundären Alkohole werden durch Reaktion der Aldehyde mit Methylgrignardverbindungen hergestellt. Die Ether sind durch Methylierung der primären Alkohole nach an sich bekannten Veretherungsverfahren zugänglich. Die Acetale sind ebenfalls nach Verfahren gemäß Stand der Technik aus den Aldehyden erhältlich.

Schließlich gelangt man durch Umsetzen von 3-Methyl- bzw. 3.5-Dimethylbenzylmagnesiumchlorid mit Aceton bzw. mit Methylethylketon zu den tertiären Alkoholen der Formel

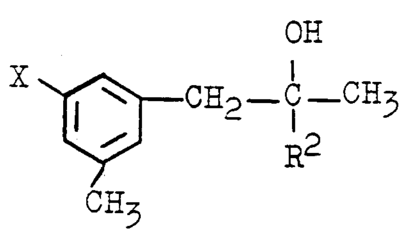

Der Reaktionsweg ist beispielhaft anhand der folgenden Reaktionsgleichung dargestellt:

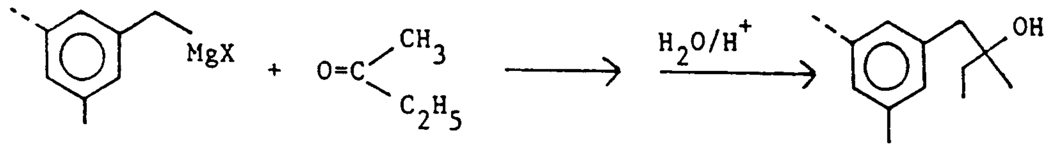


Das bevorzugte Verfahren zur Herstellung von erfindungsgemäßen Verbindungen der Formel

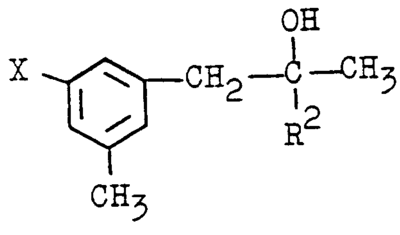

wobei

$R^2$ Ethyl und

X Wasserstoff oder Methyl

bedeuten,

ist dadurch gekennzeichnet, daß ein Benzylmagnesiumchlorid der Formel

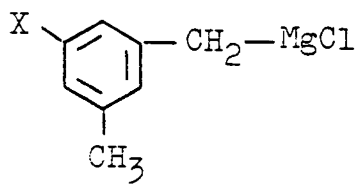

mit Methylethylketon umgesetzt wird.

Die Benzylmagnesiumchloride sind nach an sich bekannten Verfahren zur Herstellung von Grignardverbindungen aus den entsprechenden Benzylchloriden und metallischem Magnesium zugänglich. Es wird, wie üblich, in wasserfreien Lösungsmitteln, wie beispielsweise Diethylether, gearbeitet. Die Reaktionstemperaturen betragen im allgemeinen 0 °C bis 65 °C. Die anschließende saure Hydrolyse liefert den freien Alkohol. Bezüglich der Einzelheiten wird ausdrücklich auf die Beispiele verwiesen.

Die erfindungsgemäßen Verbindungen sowie 2-Methyl-3-(3-methylphenyl)-propan-2-ol und 2-Methyl-3-(3,5-dimethylphenyl)-propan-2-ol finden Verwendung als Duftstoffe. Sie werden zur Parfümierung kosmetischer und technischer Produkte eingesetzt. Es überwiegen blumige Noten, beispielsweise Noten von Maiglöckchen, Magnolien, Rosen, Cyclamen u.a.

Die Erfindung wird nun anhand von Beispielen näher erläutert:

Beispiel 1:

Herstellung von 2-(3.5-dimethylbenzyl)-3-buten-1-ol

In einem 2 1-Vierhalskolben mit Thermometer, Rührer, Tropftrichter mit Gaseinleitansatz und Rückflußkühler wurden 27 g pulverförmiges, metallisches Magnesium vorgelegt. Der Kolben wurde mit Butadien gespült und das Magnesiumpulver mit trockenem Tetrahydrofuran überschichtet. Um die Reaktion zu starten, wurden 2 ml Ethylbromid zugegeben und die Reaktionsmischung auf 55°C erwärmt. Unter ständigem Einleiten von Butadien wurden nun insgesamt 400 ml Tetrahydrofuran zugegeben. Nachdem die Reaktionsmischung eine grüngelbe Färbung angenommen hatte, wurden 155 g 3.5-Dimethylbenzylchlorid und 200 ml Tetrahydrofuran zudosiert. Die Dosierungsgeschwindigkeit wurde so geregelt, daß dabei die bereits erwähnte grüngelbe Färbung der Reaktionsmischung stets erhalten blieb. Die Butadienzufuhr wurde jeweils dem Verbrauch angepaßt. Der Verbrauch wurde anhand eines elastischen Vorratsbehälters, der am Rückflußkühler aufgesetzt war, kontrolliert. Nach 3 Stunden war das metallische Magnesium aufgelöst und

somit die erste Reaktionsstufe beendet.

Danach wurde mit Argon gespült und anschließend die Reaktionsmischung auf eine Temperatur von 0 °C abgekühlt. Zur Oxidation wurde nun trockene, $CO_2$-freie Luft eingeleitet. Der Luftstrom wurde so geregelt, daß die Temperatur nicht über 10°C stieg.

Schließlich wurde zur Hydrolyse der Kolbeninhalt unter Rühren auf Eis gegossen und mit konzentrierter Salzsäure angesäuert. Die Phasen wurden getrennt, die organische Phase noch 3 mal mit Wasser und 1 mal mit Kaliumcarbonatlösung ausgeschüttelt und schließlich mit festem Kaliumcarbonat getrocknet. Die Destillation erfolgte über eine 30 cm lange Vigreux-Kolonne. Nach dem Abdestillieren der Lösungsmittel wurden im Temperaturintervall von 80°C bis 85°C bei 0,12 mbar 78 g (3.5-dimethylbenzyl)-butenole erhalten, von denen das 2-(3.5-dimethylbenzyl)-3-buten-1-ol mit 70 % den Hauptbestandteil bildete.

Geruch: Grün-blumig, rosig mit Geraniumnote.

Beispiel 2:

Herstellung von 2-(3-methylbenzyl)-3-buten-1-ol

Es wurde die Arbeitsweise gemäß Beispiel 1 wiederholt, mit der Abänderung, daß anstatt 3.5-Dimethylbenzylchlorid 3-Methylbenzylchlorid eingesetzt wurde.

Ansatz:

35 g Magnesiumpulver

197 g 3-Methylbenzylchlorid

Es wurden durch fraktionierte Destillation bei 0,13 mbar im Temperaturintervall von 84°C bis 86°C 114 g eines 5:1 Isomerengemisches von 2-(3-Methylbenzyl)-3-buten-1-ol (als Hauptkomponente) und 1-(3-Methylphenyl)-4-penten-2-ol erhalten.

Geruch: blumig, fruchtig, grün, an Birne erinnernd.

Beispiel 3:

Herstellung von 2.2-Dimethyl-3-(3-methylphenyl)-propionaldehyd

Es wurden unter Argon 23,3 g NaOH, 2,5 g Tetrabutylammoniumjodid, 107 ml Wasser, 25 ml Toluol und 9 ml Tetrahydrofuran vorgelegt und auf 70°C erwärmt. In diese Vorlage wurde innerhalb von 12 Stunden unter kräftigem Rühren eine Mischung aus 70 g (0,5 Mol) 3-Methylbenzylchlorid und 50,4 g (0,7 Mol) Isobutyraldehyd zugetropft. Danach wurde die Reaktionsmischung noch 3 Stunden auf einer Temperatur von 75°C gehalten. Nach Phasentrennung und Abziehen der Lösungsmittel wurden durch fraktionierte Destillation bei 0,12 mbar im Temperaturintervall von 50°C bis 52°C 66,5 g an Zielprodukt erhalten.

Geruch: grün, an Laub erinnernd, mit blumig-aldehydigen Aspekten.

Beispiel 4:

Herstellung von 3-Methyl-(3-methylbenzyl)-butan-2-ol

17 g 2.2-Dimethyl-3-(3-methylphenyl)-propionaldehyd (gemäß Beispiel 3) wurden bei einer Temperatur von 25°C bis 30°C unter Luftausschluß mit einer Lösung von 10 g Methylmagnesiumchlorid in 100 ml Tetrahydrofuran versetzt. Nach 5 Stunden bei 30°C wurde die Reaktionsmischung auf Eis gegossen, mit Salzsäure angesäuert. Schließlich wurden die Phasen getrennt, die organische Phase mit 100 ml Wasser und 100 ml Natriumbicarbonatlösung gewaschen. Zum Schluß wurde mit festem Kaliumcarbonat getrocknet. Die fraktionierte Destillation ergab 15 g an Zielprodukt bei 0,12 mbar im Temperaturintervall von 79°C bis 82°C.

Geruch: Maiglöckchenduft, blumig.

Beispiel 5:

Herstellung von 2.2-Dimethyl-3-(3-methylphenyl)-propan-1-ol

Unter Argon wurden einer Vorlage aus 150 ml Isopropanol und 3,8 g Natriumborhydrid 44 g 2.2-Dimethyl-3-(3-methylphenyl)-propionaldehyd (gemäß Beispiel 3) in kleinen Portionen bei 20°C zugegeben. Es wurde noch 24 Stunden bei 20°C gerührt. Danach wurde durch Zutropfen von 2 n HCl überschüssiges

Natriumborhydrid zersetzt. Schließlich wurde die Reaktionsmischung in 150ml Diethylether aufgenommen und mit Wasser, 2 n NaOH und nochmals mit Wasser extrahiert.

Nach Trocknen über Kaliumcarbonat wurde über eine 30 cm lange Füllkörper-Kolonne destilliert. Es wurden bei 0,12 mbar im Temperaturintervall von 70°C bis 72°C 30 g an Zielprodukt erhalten.

Geruch: frischer, weicher, etwas grüner Blumenduft, an Lindenblüten und Maiglöckchen erinnernd mit Citrusnote.

Beispiel 6:

Herstellung von 1-Methoxy-2.2-dimethyl-3-(3-methylphenyl)-propan

In einen mit Argon gespülten 250 ml Dreihalskolben wurden 4,5 g 2.2-Dimethyl-3-(3-methylphenyl)-propan-1-ol (gemäß Beispiel 5) in 50 ml trockenem Tetrahydrofuran mit 0,7 g Natriumhydrid 1 1/2 Stunden unter Rückfluß gekocht. Anschließend wurden 3,2 g Dimethylsulfat in 30 ml Tetrahydrofuran zugetropft und die Mischung eine weitere Stunde am Rückfluß gekocht. Nach 24-stündigem Stehenlassen wurde das Reaktionsgemisch mit 20 ml 10 Gew.-%iger Natronlauge ausgeschüttelt. Schließlich wurde die organische Phase mit Kaliumcarbonat getrocknet und fraktioniert destilliert. Bei 52°C/0,12 mbar wurden 3,6 g an Zielprodukt erhalten.

Geruch: würzig-blumig mit frischer Kopfnote.

Beispiel 7:

Analog zu Beispiel 6 wurden 17,6 g 2-(3-Methylbenzyl)-3-buten-1-ol (gemäß Beispiel 2) in 80 ml Tetrahydrofuran mit 2,6 g Natriumhydrid umgesetzt und das entstandene Alkoholat mit 12,6 g Dimethylsulfat in 100 ml Tetrahydrofuran zur Reaktion gebracht.

Die fraktionierte Destillation ergab bei 60°C/0,25 mbar 10 g an Zielprodukt.

Geruch: süß holzig, leicht blumig, grün.

Beispiel 8:

Herstellung von 2.2-Dimethyl-3-(3.5-dimethylphenyl)-propionaldehyd

Eine Vorlage aus 125 g 30 Gew.-%iger Natronlauge, 5 g Kaliumjodid, 10 g Tetradecyltrimethylammoniumbromid, 200 ml Tetrahydrofuran und 200 ml Toluol wurde auf 75°C erwärmt. In diese Vorlage wurde unter kräftigem Rühren eine Mischung aus 155 g 3.5-Dimethylbenzylchlorid, 110 g Isobutyraldehyd, 50 ml Tetrahydrofuran und 50 ml Toluol getropft. Danach wurde noch 6 Stunden am Rückfluß gekocht. Danach wurden die Phasen getrennt und die organische Phase zunächst mit warmen Wasser gewaschen und anschließend unter Zusatz von Aktivkohle filtriert. Schließlich wurde über eine Füllkörper-Kolonne mit Drahtwendeln destilliert. Die Destillation ergab im Temperaturintervall von 72°C bis 74°C bei 0,25 mbar 106 g an 92 %igem 2.2-Dimethyl-3-(3.5-dimethylphenyl)-propionaldehyd.

Geruch: Grünnote, leicht blumig, aldehydig, würzig, etwas an Safrol erinnernd.

Beispiel 9:

Herstellung von 2.2-Dimethyl-3-(3.5-dimethylphenyl)-propan-1-ol

Zu einer mit Argon gespülten Vorlage von 3 g Natriumborhydrid in 100 ml Isopropanol in einem 500 ml Vierhalskolben wurden im Temperaturbereich von 30°C bis 35°C 20 g 2.2-Dimethyl-3-(3.5-dimethylphenyl)-propionaldehyd getropft. Nach 2 Stunden wurde die Temperatur des Reaktionsgemisches noch für 1 Stunde auf 60°C erhöht. Danach wurde überschüssiges Natriumborhydrid durch Zugabe von 2 n Salzsäure zersetzt. Anschließend wurde die Hauptmenge an Isopropanol abdestilliert und der Rückstand in 100 ml Ether aufgenommen. Schließlich wurde noch mit 2 n NaOH und Wasser extrahiert und die organische Phase mit Kaliumcarbonat getrocknet. Die fraktionierte Destillation erbrachte im Temperaturbereich von 81°C bis 83°C bei 0,12 mbar 9 g an Zielprodukt.

Geruch: Duft von weißen Blüten, Maiglöckchen, Magnolien,Tuberose,zudem etwas ambriert.

Beispiel 10:

Herstellung von 2-(3.5-Dimethylbenzyl)-butan-2-ol

Aus 2,6 g Magnesiumpulver und 15,5 g 3.5-Dimethylbenzylchlorid, gelöst in 200 ml Diethylether, wurde 3.5-Dimethylbenzylmagnesiumchlorid hergestellt und im Temperaturbereich von 20°C bis 25°C mit 7,5 g Methylethylketon umgesetzt. Nach 2 Stunden wurde der Ansatz auf Eis gegossen, angesäuert, die organische Phase abgetrennt und mit Wasser und Natriumbicarbonatlösung ausgeschüttelt. Nach Trocknen der organischen Phase mit festem Kaliumcarbonat wurde fraktioniert destilliert. Im Temperaturintervall 63°C bis 65°C wurden bei 0,12 mbar 13 g an Zielprodukt erhalten.

Geruch: grün, an Cyclamen und Maiglöckchen erinnernd, mit Grünnote von Laub.

Beispiel 11:

Herstellung von 2-Methyl-2-(3-methylbenzyl)-butyraldehyd

In einem 1 l Dreihalskolben wurden 300 ml Toluol, 70 g 30 Gew.-%ige Natronlauge, 3,3 g Kaliumjodid und 6,7 g Tetradecyltrimethylammoniumbromid vorgelegt. In diese Vorlage wurde innerhalb einer 1/2 Stunde bei 85°C eine Mischung aus 70 g 3-Methylbenzylchlorid, 50 ml 2-Methylbutyraldehyd und 100 ml Toluol getropft. Danach wurde das Reaktionsgemisch bei 85°C noch 4 Stunden gerührt. Nach Trennen der Phasen wurde die organische Phase mehrmals mit Wasser gewaschen und über Aktivkohle filtriert. Schließlich wurde fraktioniert destilliert. Es wurden bei 63°C/0,06 mbar 40 g eines Produkts erhalten mit einem Gehalt von 93 % an Zielprodukt.

Geruch: Grünnote mit ozonig-blumigen Charakter, etwas würzig.

Beispiel 12:

Herstellung von 2-Methyl-2-(3-methylbenzyl)-butan-1-ol

29 g 2-Methyl-2-(3-methylbenzyl)-butyraldehyd (gemäß Beispiel 11) wurden mit 3 g Natriumborhydrid in 80 ml Ethanol 20 Stunden bei Raumtemperatur gerührt. Danach wurde überschüssiges Natriumborhydrid mit 2 n HCl zersetzt. Nach Abziehen des Ethanols wurde der Rückstand in Ether aufgenommen und mit Wasser extrahiert. Die organische Phase wurde noch mit 2 n NaOH und Wasser ausgeschüttelt und mit festem Kaliumcarbonat getrocknet. Nach Abziehen des Lösungsmittels wurde fraktioniert destilliert. Im Temperaturbereich von 72°C bis 73°C bei 0,12 mbar wurden 20 g an Zielprodukt erhalten.

Geruch: frische Blumennote, etwas fruchtig, holzig.

## Patentansprüche

1. Verbindungen der allgemeinen Formel (I)

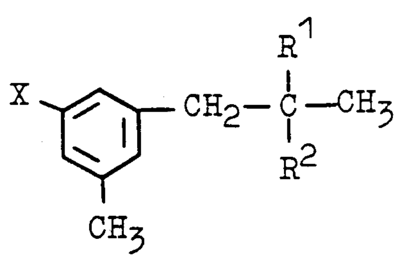

und Verbindungen der Formel (II)

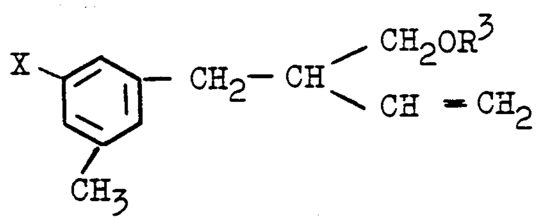

wobei

$R^1$ für

-OH, $-CH_2OH$, $CH_2-OCH_3$, $-CHOH-CH_3$,
-CHO und $-CH(OCH_3)_2$

steht,

$R^2$ Methyl oder Ethyl

$R^3$ Wasserstoff oder Methyl und

X Wasserstoff oder Methyl

bedeuten und 2-Methyl-3-(3-methylphenyl)-propan-2-ol und 2-Methyl-3-(3,5-dimethylphenyl)-propan-2-ol ausgeschlossen sind.

**2.** Verfahren zur Herstellung von Verbindungen der Formel

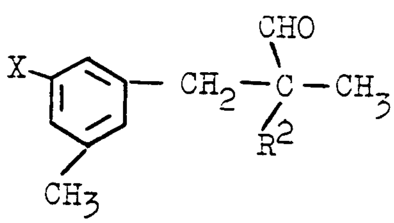

wobei

$R^2$ Methyl oder Ethyl und

X Wasserstoff oder Methyl

bedeuten, dadurch gekennzeichnet, daß Benzylchloride der Formel

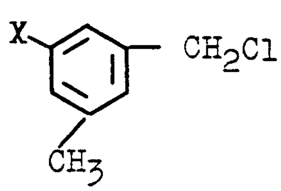

mit Isobutyraldehyd oder 2-Methylbutyraldehyd in einem organisch/alkalischen 2-Phasensystem, ggf. in Gegenwart von Phasentransferkatalysatoren, kondensiert werden.

**3.** Verfahren zur Herstellung der Verbindungen gemäß Formel (II), dadurch gekennzeichnet, daß
a) Benzylchlorid der Formel

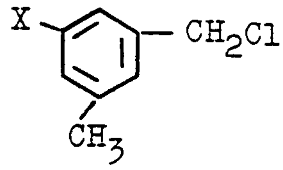

wobei
X für Wasserstoff oder Methyl steht,
mit Butadienmagnesium umgesetzt wird,
b) das Reaktionsprodukt gemäß a) oxidiert wird,
c) das Oxidationsprodukt gemäß b) einer sauren Hydrolyse unterworfen wird,
d) das Reaktionsprodukt gemäß c) ggf. an der freien OH-Gruppe methyliert wird.

**4.** Verfahren zur Herstellung der Verbindungen der allgemeinen Formel

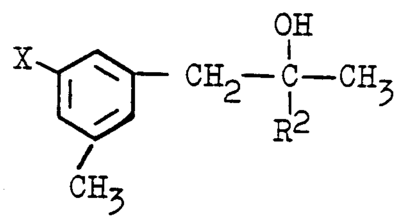

wobei
$R^2$ Ethyl und
X Wasserstoff oder Methyl
bedeuten, dadurch gekennzeichnet, daß ein Benzylmagnesiumchlorid der Formel

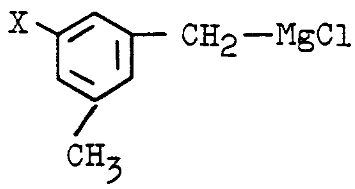

mit Methylethylketon umgesetzt wird.

**5.** Verwendung der Verbindungen nach Anspruch 1 sowie von 2-Methyl-3-(3-methylphenyl)-propan-2-ol und 2-Methyl-3-(3,5-dimethylphenyl)-propan-2-ol als Duftstoffe.

## Claims

**1.** Compounds of the general formula (I)

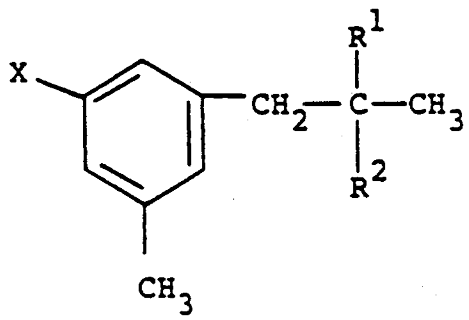

and compounds of the formula (II)

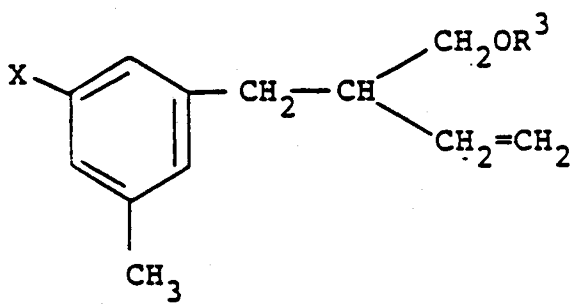

in which

- $R^1$ represents -OH, $-CH_2OH$, $CH_2-OCH_3$, $-CHOH-CH_3$, -CHO and $-CH(OCH_3)_2$,
- $R^2$ represents methyl or ethyl,
- $R^3$ represents hydrogen or methyl and
- X represents hydrogen or methyl,

and 2-methyl-3-(3-methylphenyl)-propan-2-ol and 2-methyl-3-(3,5-dimethylphenyl)-propan-2-ol are excluded.

**2.** Process for the manufacture of compounds of the formula

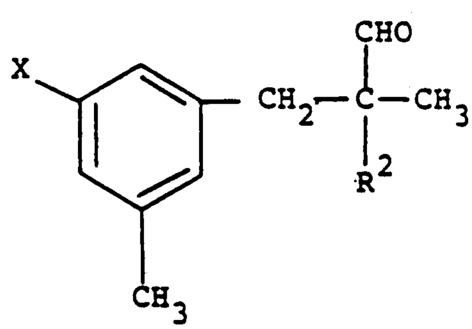

in which

- $R^2$ represents methyl or ethyl and
- X represents hydrogen or methyl,

characterised in that benzyl chlorides of the formula

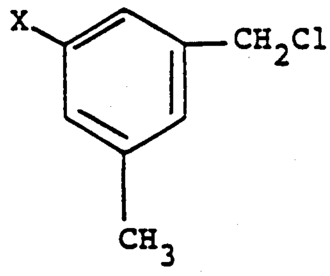

are condensed with isobutyraldehyde or 2-methylbutyraldehyde in an organic/alkaline 2-phase system, optionally in the presence of phase transfer catalysts.

**3.** Process for the manufacture of the compounds according to formula (II), characterised in that
a) benzyl chloride of the formula

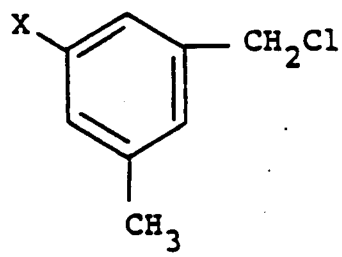

in which
X represents hydrogen or methyl,
is reacted with butadienemagnesium
b) the reaction product according to a) is oxidised,
c) the oxidation product according to b) is subjected to acidic hydrolysis,
d) the reaction product according to c) is optionally methylated at the free OH group.

**4.** Process for the manufacture of the compounds of the general formula

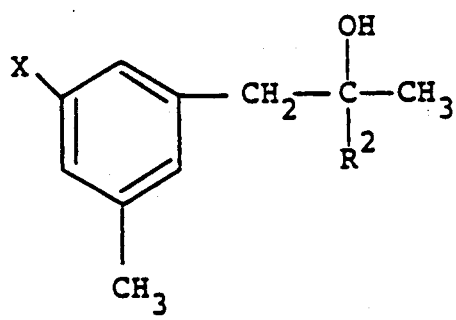

in which
$R^2$ represents ethyl and
X represents hydrogen or methyl,
characterised in that a benzylmagnesium chloride of the formula

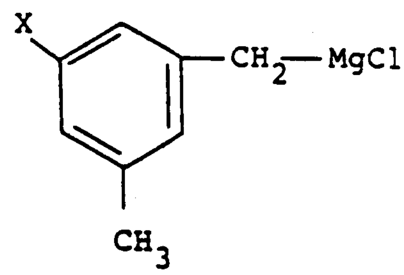

is reacted with methyl ethyl ketone.

**5.** Use of the compounds according to claim 1 as well as 2-methyl-3-(3-methylphenyl)-propan-2-ol and 2-methyl-3-(3,5-dimethylphenyl)-propan-2-ol as perfume materials.

**Revendications**

**1.** Composés de formule générale (I)

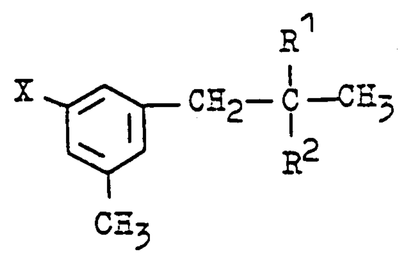

et composés de formule (II)

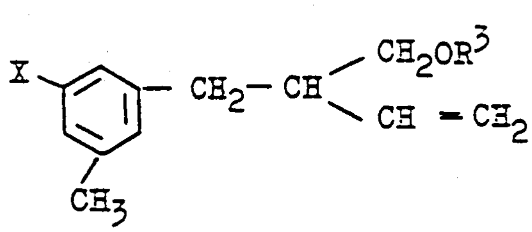

où

$R^1$ est -OH, $-CH_2OH$, $-CH_2-OCH_3$, $-CHOH-CH_3$, -CHO et $-CH(OCH_3)_2$

$R^2$ est le radical méthyle ou éthyle,

$R^3$ est un hydrogène ou le radical méthyle, et

X est un hydrogène ou le radical méthyle,

à l'exclusion des composés méthyl-2 (méthyl-3 phényl)-3 propanol-2 et méthyl-2 (diméthyl-3,5 phényl)-3 propanol-2.

**2.** Procédé de préparation de composés de formule

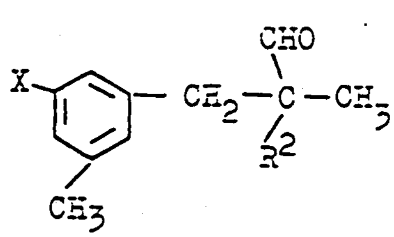

dans laquelle $R^2$ est le radical méthyle ou éthyle, et X est un hydrogène ou le radical méthyle, caractérisé en ce qu'on condense des chlorures de benzyle de formule

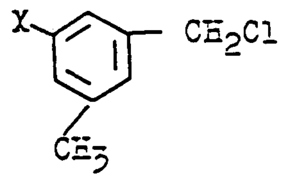

avec de l'isobutyraldéhyde ou du méthyl-2 butyraldéhyde dans un système biphasique organique/alcalin, éventuellement en présence de catalyseurs de transfert de phase.

**3.** Procédé de préparation des composés de formule (II), caractérisé en ce que
a) On fait réagir un chlorure de benzyle de formule

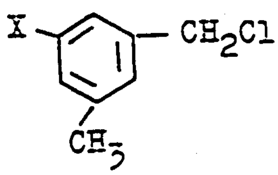

dans laquelle X est un hydrogène ou le radical méthyle, avec du butadiène-magnésium,
b) On oxyde le produit de réaction selon a),
c) On soumet à une hydrolyse acide le produit d'oxydation selon b),
d) On méthyle le produit de réaction selon c), éventuellement au niveau du groupe OH libre.

**4.** Procédé de préparation des composés de formule générale

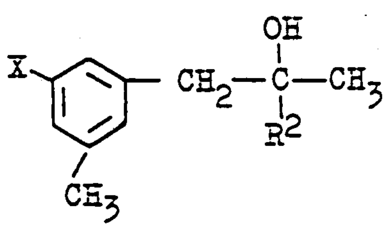

dans laquelle $R^2$ est le radical éthyle et X est un hydrogène ou un radical méthyle, caractérisé en ce qu'on fait réagir avec de la méthyléthylcétone un chlorure de benzylmagnésium de formule

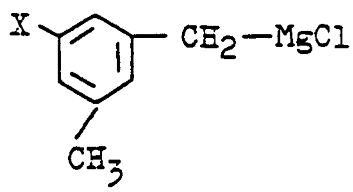

**5.** Utilisation des composés selon la revendication 1, ainsi que du méthyl-2 (méthyl-3 phényl)-3 propanol-2 et du méthyl-2 (diméthyl-3,5 phényl)-3 propanol-2 en tant que parfums.